(19) Europäisches Patentamt
European Patent Office
Office européen des brevets

(11) Publication number: **0 003 872**
**A1**

(12) # EUROPEAN PATENT APPLICATION

(21) Application number: 79300159.5

(22) Date of filing: 31.01.79

(51) Int. Cl.²: C 07 C 101/62
C 08 F 2/50, C 08 K 5/18

(30) Priority: 16.02.78 GB 624578

(43) Date of publication of application:
05.09.79 Bulletin 79/18

(84) Designated contracting states:
BE CH DE FR GB IT NL SE

(71) Applicant: WARD BLENKINSOP AND COMPANY LIMITED
Fulton House Empire Way
Wembley, Middlesex HA9 0LX (GB)

(72) Inventor: Birchwood, Peter John
Fulton House Empire Way
Wembley Middlesex HA9 0LX (GB)

(72) Inventor: Green, Peter Nicholl
Fulton House Empire Way
Wembley Middlesex HA9 0LX (GB)

(72) Inventor: Young, Joseph Robert Alan
Fulton House Empire Way
Wembley Middlesex HA9 0LX (GB)

(74) Representative: Pennant, Pyers et al,
Stevens, Hewlett & Perkins 5 Quality Court Chancery Lane
London, WC2A 1HZ (GB)

(54) **Substituted benzoic acid esters, compositions containing them, combinations thereof with photoinitiators and methods of making cured plastics compositions using them.**

(57) Para-dialkylaminobenzoic acid esters of the formula:

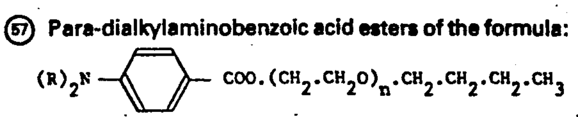

Where R is methyl or ethyl and n = 1 or 2 are described. These compounds are potent photoactivators especially for photoinitiators of the substituted thioxanthone type. All the compounds of the invention are liquids at temperature down to 0°C thus markedly facilitating compounding. Rapidly curing photocurable compositions e.g. film of $TiO_2$ pigmented photocurable resins can be formulated such as can be used as or in printing inks.

EP 0 003 872 A1

Croydon Printing Company Ltd.

# Substituted benzoic acid esters, compositions containing them combinations thereof with photoinitiators and methods of making cured plastics compositions using them

This invention relates to substituted benzoic acid esters, and in particular to their use as photoactivators, to photocurable compositions containing them as photoactivators, to combinations of such photoactivators and photoinitiators and to their use in photocuring resins especially pigmented resins and in particular to resins pigmented with titanium dioxide.

Photoactivators are compounds, usually organic compounds, which form synergistic combinations with photoinitiators in the photocuring of ethylenically unsaturated resins. In the photocuring of such resins the photoinitiator is a compound which will absorb light energy from the curing radiation to give an energetic species which can start a chain reaction to cross-link and thus cure the resin. It is believed that photoactivators take part in the chain by having a proton abstracted by the energetic species of the photoinitiator to produce a free radical which can subsequently initiate cross-linking.

Most photoinitiators in current use are ketones or ketone derivatives. Typical examples are acetophenone, benzophenone, benzil, benzoin, xanthone, thioxanthone and substituted forms and derivatives of these

compounds. 2-Substituted thioxanthones, especially 2-chlorothioxanthone (2-CTX) and the 2-alkylthioxanthones are particularly useful where $TiO_2$ pigments are used because they have a photoactive absorption band centred about 380 nm and this is in the transitional region between the lower wavelength end of the high reflectivity and the upper wavelength end of the U.V. absorption band of $TiO_2$.

Typically the photoactivators in current use are amines and in particular tertiary amines. Typical examples include triethylamine, triethanolamine, methyldiethanolamine, dimethylaniline and morpholine. Recently the use of para-dialkylamino benzoic acid and its esters has been suggested in German Offenlegungsschrift No. 2458345.

One disadvantage of 2-chloro and 2-alkyl thioxanthones is that they are solids. Since in most photocuring systems it is preferred to avoid the introduction of solvent which would have to be subsequently removed from the system, those photoinitiators have to be milled into the composition to be photocured. The lower members of the homologous series of para-dialkylaminobenzoate esters are also all solids and this constitutes a substantial disadvantage of what would otherwise be very attractive photoactivators.

The present invention accordingly provides a compound of the formula:

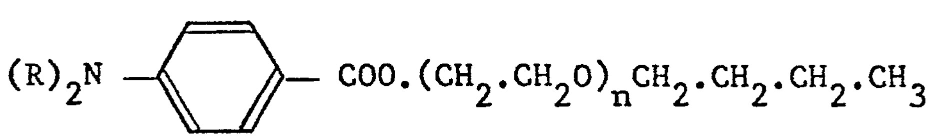

where each R is a methyl or ethyl group and n is 1 or 2. The invention includes the compound 2-(n-butoxy)ethyl 4-dimethylaminobenzoate.

The invention also provides a photocurable composition comprising a photocurable ethylenically unsaturated compound, a photoinitiator and as a photoactivator one or more compounds of the invention.

The invention further includes a method of making a cured plastics composition which comprises exposing a composition of the invention to a dose of active radiation sufficient to cure it.

The photoinitiators used in this invention are typically di-aryl ketones. This type of photoinitiator is commonly used with photoactivators in particular when used in pigmented resin systems. The simplest di-aryl ketones photoinitiators are those of the benzophenone type, the most commonly used compound being benzophenone itself. The photoactivators used in the present invention can be used with benzophenone but we have obtained better results from di-aryl ketone photoinitiators in which the two aryl groups are linked to give a cyclic ketone. Examples of such ketone photoinitiators include 2-substituted thioxanthones, especially 2-chloro- and 2-alkyl- thioxanthones, dibenzosuberone and fluorenenone. We have found that benzil photoinitiators also work with the photoactivators used in the present invention and for the purposes of this invention we include benzil i.e. 1,2-diphenylethane-1,2-dione and substituted benzils, but not functional derivatives of the oxo-groups, within the term di-aryl ketone.

We believe that this is well understood by those skilled in the art. The invention includes a composition comprising a photoinitiator and as a photoactivator a compound of the formula:

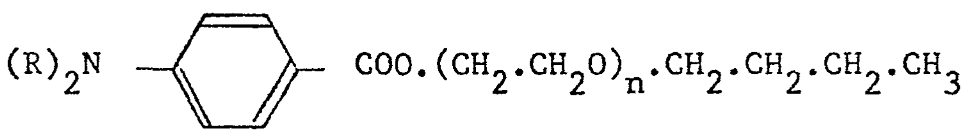

where R and n are as defined above.

It is a particular advantage of the compounds used as photoactivators in the present invention that they are liquids not only at ambient temperatures but at temperatures down to $0^{o}C$, and that at least the most commonly used 2-alkylthioxanthones, 2-isopropylthioxanthone and 2-tertiarybutylthioxanthone are readily soluble, e.g. up to 40 g. per 100 g. ester and 2-chlorothioxanthone has a significant solubility sufficient that in curing resins the necessary 2-CTX can be completely dissolved in the ester. Accordingly, in a preferred aspect the invention further provides a photoinitiator-photoactivator combination comprising a compound of the formula:

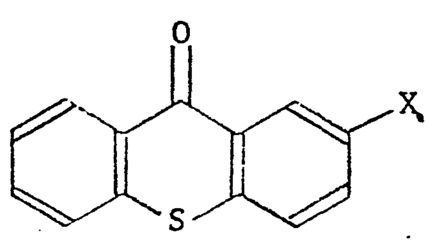

where X is a chlorine atom (giving 2-CTX) or an alkyl group (giving the 2-alkylTX's), preferably a $C_1$ to $C_{10}$ alkyl group, more preferably a branched chain $C_3$ to $C_7$ alkyl group, optimally an isopropyl or tertiary-butyl group; in solution in a compound or a mixture of compounds of the formula:

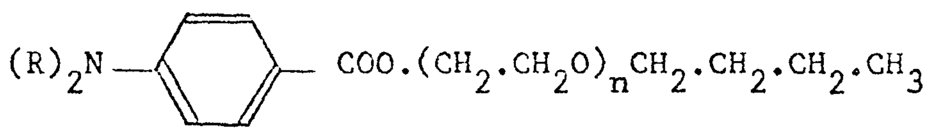

where R and n are as defined above.

The compounds of the invention can be used as photoactivators in the photocuring of plastics compositions. For example, they are particularly useful in the manufacture of cured thin plastics films especially printing inks.

The photoactivators are useful generally in the photocuring of resins containing cross-linkable ethylenically unsaturated compounds. Typically the ethylenic unsaturations are provided by including oligomeric olefins or unsaturated polyesters, e.g. those made including an unsaturated acid such as maleic, fumaric or itaconic acid. The unsaturation may be introduced into the resin either by copolymerising a suitable ethylenically unsaturated monomer with the other components of the resin or by mixing a separately formed polymer containing ethylenic unsaturations with a polymer containing no ethylenic unsaturations. The choice in any particular case will depend upon the

desired properties in the end product. Suitable polymer resins are acrylic resins, acrylated aromatic or aliphatic polyurethane resins, epoxy acrylate resins and styrene polyester resins.

The amount of photoactivator used depends on the nature of the resins, the amount and nature of the pigment and the particular photoinitiator used. Typically to ensure adequate photoactivation the amount used will be from 0.2 to 5%, preferably 1 to 3%, by weight of the composition. The amount of photoinitiator depends on the particular photoinitiator employed, but will typically be in the range 0.1 to 5% by weight of the composition. When 2-CTX or 2-alkylTX is used as the photoinitiator, the amount used will usually be in the lower part of this range, e.g. 0.2 to 1, preferably 0.2 to 0.5. These figures represent practical levels of photoactivator and photoinitiator in $TiO_2$ pigmented systems even at high pigment levels, e.g. up to 50% by weight of the composition. When other pigments are used the photoinitiator will be selected accordingly but this is regarded as within the skill in the art. The ratio between the photoactivator and photoinitiator is not believed to be especially critical but is generally in the range 1:4 to 100:1. Especially when 2-CTX is the photoinitiator it is preferable that this ratio is fairly high, e.g. in the range 4:1 to 20:1 because the use of large quantities of 2-CTX tends to produce a yellow colour in the cured resin. The photoactivators used in the present invention are sufficiently potent that such low levels, both relatively and absolutely,

of 2-CTX can still produce good results.

Where, as is preferred, the photoactivator-photoinitiator combination is a solution of 2-CTX or 2-alkyl-TX in the benzoate ester, the amount of this combination used will typically be from 1 to 5% by weight of the composition. The solution will typically contain from 1 to 20% by weight of the photoinitiator. However, where 2-chlorothioxanthone is the photoinitiator the concentration will generally be in the range of 1 to 5% because of the rather limited solubility of the commercially available forms of 2-CTX. Technical grade 2-CTX available at the present time typically contains about 65% by weight of 2-chlorothioxanthone and about 35% by weight of 3-chlorothioxanthone which is somewhat less active that the 2-isomer. This material is soluble in 2-(n-butoxy)ethyl p-dimethylaminobenzoate to about 5 % $^{w}/w$.

The resins used in the invention are generally photocured by exposure to ultra violet or short wavelength visible light, the precise wavelength or range of wavelengths depending on the pigment and photoinitiator used. Where, as is preferred in the present invention, the pigment is $TiO_2$, the light used will generally be predominantly ultraviolet light in the wavelength range 350 to 400 nm. For other pigments other wavelengths are appropriate as is known in the art. The time of exposure depends inter alia on the intensity of the radiation, the thickness of the resin to be cured and the nature and concentration of the photoinitiator and photoactivator. We have found that

in the present invention exposures of less than ½ sec. using a single lamp (80 W $cm^{-1}$) Minicure oven for thin films of $TiO_2$ pigmented resin are adequate to give hard (> 6H pencil hardness) layer of cured pigmented resin. The use of thicker layers of resin or lower radiation intensity will generally require longer exposure times.

The compounds of the present invention have activity as photoactivators which is comparable to that of known photoactivators such as the photoactivators described in our U.K. Patent Specification No. (Application No. 52202/76). The low melting points of the compounds of the invention provides a particular advantage in that in combination with their solubility for the normal photoinitiators it enables the user to add the active ingredients in a liquid form after the milling of the pigment into the resin so reducing wastage. The paradimethylaminobenzoic acid esters whose use as photoactivators is described in German Offenlegungsschrift No. 2458345 have, as a class, substantially higher melting points than the compounds of the present invention. Typically they are solid rather than liquid at ambient temperature. Their generally solid nature makes them substantially more difficult to compound with resins than the compounds and compositions of the present invention.

The possibility of incorporating pigments into the resin products has been mentioned above. The invention is applicable to cured resin products containing no pigment and to those containing pigments which may be white e.g. titanium dioxide or coloured. The invention

is also applicable to products which are clear but are coloured by containing a soluble dyestuff. Of course, where pigmented or dyed products are being produced the precise conditions of operation may need to be adjusted in order to take account of the possibility of dispersion or adsorption of the curing radiation by the pigment of dyestuff. This is a problem that is well understood in the art and no particular difficulty arises in dealing with it in the present invention.

The compounds of the invention can be made by methods which are known or analagous to methods known per se for similar compounds. It is an additional advantage of the compounds of the invention that they can readily be made from compounds which are already commercially available. The following reaction scheme illustrate some ways in which the compounds of the invention can be made. Full experiment details are not included in these schemes because these are either readily found or known from the literature.

REACTION SCHEME

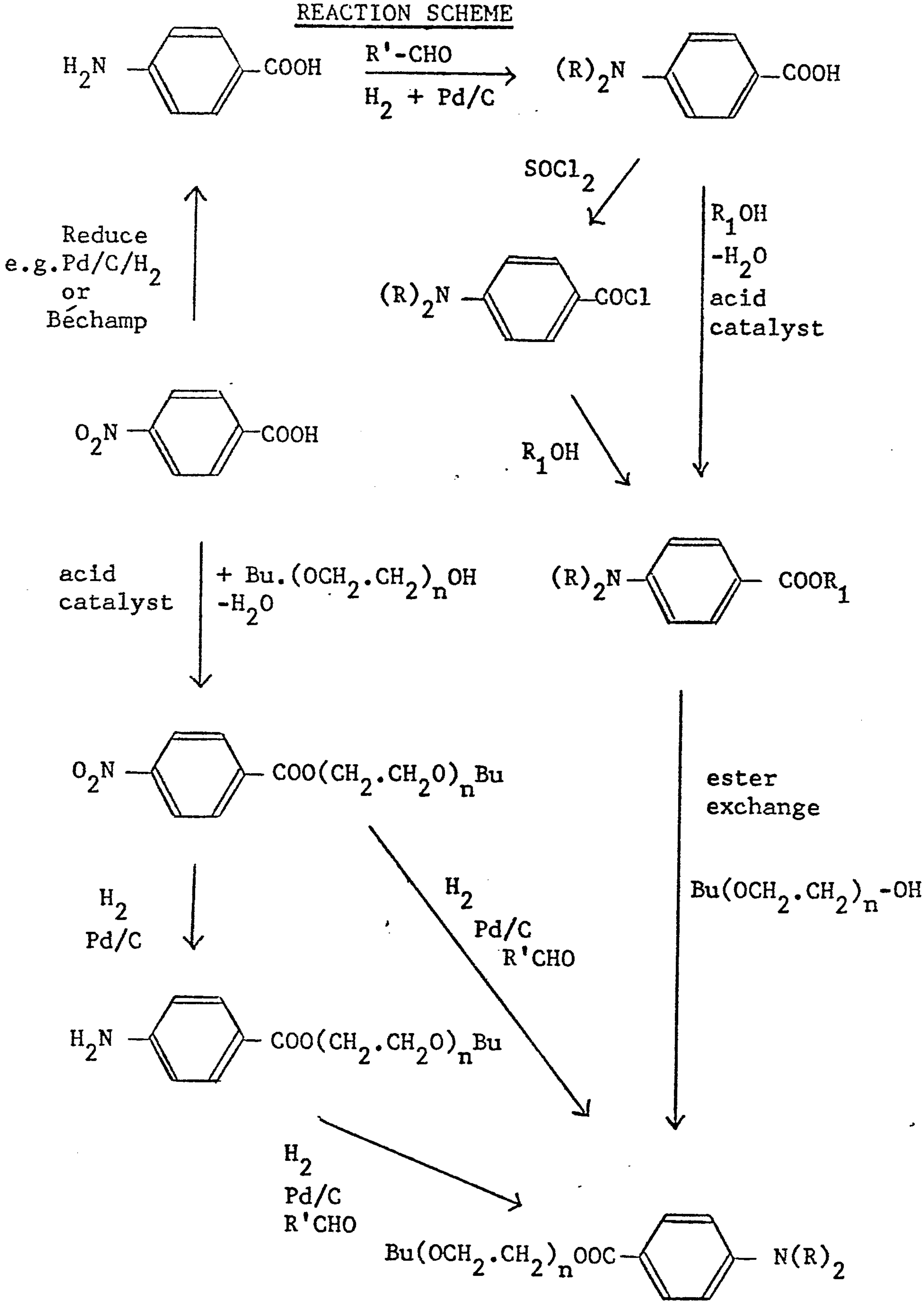

In the above reaction scheme R and n have the meanings given above; R' is hydrogen or methyl such that $R'CH_2$ is the same as R; $R^1$ is a group suitable for ester exchange e.g. a lower alkyl group such as methyl or ethyl; and Bu represents a butyl group.

The following Examples illustrate the invention. In these Examples Uvimer D.V. 530 resin was used. This resin is a commercially available acrylated aromatic polyurethane resin. The grade of titanium dioxide pigment used in the Examples is R-TC2 grade. All film samples were cured by exposing for the stated time using a single lamp (80 $W.cm^{-1}$) Minicure oven.

## EXAMPLE 1

## 2-n-Butoxyethyl 4-dimethylaminobenzoate

### Preparation

30 g. of ethyl p-dimethylaminobenzoate (0.155M), 25 mls. of 2-butoxyethanol (22.5 g., 0.19M) and 90 mls. of dry toluene were refluxed together until all extraneous water present has been removed via a partial take-off head. The solution was allowed to cool below boiling point, 1 g. of dry sodium methoxide was added and the solution refluxed for four hours. Fractions of boiling point between 76° and 94° were run off until the temperature at the still head was a constant 100°C. The solution was then allowed to cool below the boil, a further 1 g. of sodium methoxide added, and refluxed overnight, fractions being run off until a constant temperature of 108-100° was achieved at the still head.

The solution was cooled, filtered, washed with 80 mls. water the organic layer separated and dried with sodium sulphate, filtered and rotary evaporated to give a pale yellow liquid, 93.2% pure by non-aqueous titration (30.8 g corresponding to 69.8% yield).

On high vacuum distillation, a colourless liquid of boiling point 178-181°C/1.25 - 1.5 mm/Hg. was obtained. Assay (non-aqueous titration) 99.5%, M.Pt. -1.0°C.

T.L.C. $R_f$ = 0.31 ($CHCl_3/SiO_2$)

Microanalysis

| % Wt. | $C_{15}$ | $H_{23}$ | N | $O_3$ |
|---|---|---|---|---|
| Theoretical | 67.90 | 8.74 | 5.28 | 18.09 |
| Found | 68.14 | 8.64 | 5.30 | 17.92 (by difference) |

Photoactivator activity

Experiment 1

4% w/w of a mixture of equal parts of n-butoxyethyl 4-dimethylaminobenzoate and 2-chlorthioxanthone (2-CTX) was added to 50% w/w $TiO_2$ pigmented Uvimer D.V. 530 resin and the mixture was then stirred at 60°C for five minutes. A 25 micron (1/1000") thick film of the cooled mixture was made on a glass slide and U.V. irradiated. A pale yellow high gloss film of pencil hardness greater than 6H was obtained in 0.05 seconds.

Experiment 2

The solubility of isopropylthioxanthone in n-butoxyethyl 4-dimethylaminobenzoate was found to be 29.4% at room temperature. A 13% w/w solution was made up and 2.5% w/w of the latter was incorporated into the same resin-pigment composition used in Experiment 1. A very pale yellow high gloss film of pencil hardness

greater than 6H was obtained in 0.15 seconds. The melting point of the 13% w/w solution was found to be $-8^{o}C$.

EXAMPLE 2

2-n-Butoxyethyl 4-diethylaminobenzoate

Preparation

Ethyl p-diethylaminobenzoate (20 g.) n-butoxyethanol (13 mls.), sodium methoxide (1 g.) and dry toluene were refluxed together for 4 hours, fractions of boiling point 76-100$^{o}$C being run off via a partial take-off head until a constant still head temperature of 108$^{o}$C was achieved. The refluxing mixture was allowed to go off the boil and a further 1 g. of sodium methoxide was added followed by refluxing for a further 12 hours until a constant still head temperature of 108$^{o}$C was achieved. The mixture was cooled, filtered, washed with 50 mls. water, the organic layer separated and dried with sodium sulphate, filtered and rotary evaporated to give a pale yellow liquid, 75.0% pure by g.l.c. (15.2 g. corresponding to a yield of 43.0%). Further purification was effected by column chromatography ($CH_2Cl_2/SiO_2$) giving n-butoxyethyl 4-diethylaminobenzoate as a colourless liquid, 99.3% pure by g.l.c. M.Pt. = below -20$^{o}$C.

T.L.C. $R_f$ = 0.29 ($CHCl_3/SiO_2$)

Microanalysis

| % Wt. | $C_{17}$ | $H_{27}$ | N | $O_3$ |
|---|---|---|---|---|
| Theoretical | 69.59 | 9.28 | 4.77 | 16.36 |
| Found | 69.91 | 9.11 | 4.83 | 16.15 (by difference) |

Photoactivator Activity

4% w/w of a mixture of equal parts of n-butoxyethyl 4-diethylaminobenzoate and 2-chlorothioxanthone was incorporated into the same resin pigment composition used in Example 1. A pale yellow high gloss film of pencil hardness greater than 6H was obtained in 0.075 seconds. The colour of the film was less yellow than that obtained in Experiment 1 of Example 1.

EXAMPLE 3

n-Butoxyethoxyethyl 4-dimethylaminobenzoate

Preparation

Ethyl p-dimethylaminobenzoate (30 g.), butyl diethoxol (25 mls.), sodium methoxide (1 g.) and toluene (150 mls.) were refluxed together for four hours, fractions of boiling point 76-100°C being run off using a partial take off head. The mixture was allowed to cool below the boil, further sodium methoxide (1 g.) and butyl diethoxol (15 mls.) added and the mixture refluxed for a further two days, fractions being run off until a constant still head temperature of 108°C was achieved. The mixture was cooled to room temperature, filtered and rotary evaporated and impurities were removed by high vacuum distillation leaving a yellow oil of boiling point 66-74°C/0.8 mm. 97.6% pure by g.l.c., 103.3% by non-aqueous titration. (36.5 g. corresponding to 74.4% yield). M.Pt. below -10°C. T.L.C. $R_f$ = 0.22 ($CHCl_3/SiO_2$).

Microanalysis

| % Wt. | $C_{17}$ | $H_{27}$ | N | $O_4$ |
|---|---|---|---|---|
| Theoretical | 65.99 | 8.80 | 4.53 | 20.68 |
| Found | 66.13 | 8.80 | 4.60 | 20.47 (by difference) |

Photoactivator Activity

4% w/w of a mixture of equal parts of n-butoxyethoxyethyl 4-dimethylaminobenzoate and 2-chlorothioxanthone was incorporated into the same resin pigment composition used in Example 1. A pale yellow high gloss film of pencil hardness greater than 6H was obtained in 0.05 seconds. The colour of the film was similar to that obtained in Experiment 1 of Example 1.

EXAMPLE 4

2-n-Butoxyethyl p-dimethylaminobenzoate

Alternative Synthesis

(i) Preparation of 2-n-butoxyethyl p-nitrobenzoate

200 g. of p-nitrobenzoic acid (1.2M), 141 g. of 2-butoxyethanol (1.2M) and 5 g. of p-toluenesulphonic acid were stirred together in 500 ml. toluene. The mixture was refluxed for 20 hours, removing the water formed azeotropically. The volume of water collected was 22 ml. The mixture was cooled to 20$^{o}$, treated with water (225 ml.) followed by sodium bicarbonate (15 g.) in portions until the pH was 8.0-8.5. The mixture was allowed to settle and the lower aqueous layer separated. The upper toluene layer was evaporated under reduced pressure to give crude title compound.

Yield = 332 g. = 103.8%

(Assay (g.l.c.) = 93%)

The compound was found to be sufficiently pure to be used in the rest of the synthesis without specific purification.

(ii) Preparation of 2-n-butoxyethyl p-dimethylaminobenzoate

Two routes for the conversion of 2-n-butoxyethyl p-nitrobenzoate to the title compound were tried.

a) Single stage method

100 g. of crude 2-n-butoxyethyl p-nitrobenzoate, 300 ml. methanol, 100 ml. 40% formalin, 6 ml. acetic acid and 4 g. of Pd/C paste were charged to a stainless steel hydrogenator. The hydrogenator was sealed and the mixture hydrogenated at 490 $KN/m^2$ (70 p.s.i.) and 75-80°C for 24 hours. After cooling to 55°, the excess pressure was vented and the mixture filtered to recover catalyst, washing with methanol (2 x 25 ml.). The filtrate was treated with water (300 ml.), and the precipitated oil extracted into toluene (200 ml.). The lower aqueous phase was separated and the toluene layer treated with water (100 ml.) and sodium bicarbonate (ca. 2 g.) to give a pH of 8.0-8.5. The lower aqueous phase was separated and the toluene layer treated with activated charcoal (2 g.), stirred at 20-25° filtered and the filtrate evaporated to dryness under reduced pressure.

Yield = 92.5 g.
= 96.8% based on p-nitrobenzoic acid.

Assay (g.l.c.) = 96.8%

b) Two stage method

100 g. of crude 2-butoxyethyl p-nitrobenzoate, methanol (150 ml.) and Pd/C paste (2 g.) were charged to a stainless steel hydrogenator and hydrogenated at 490 $KN/m^2$ (70 p.s.i.) and 75-80°C for 18 hours. After cooling to 55° the mixture was filtered to recover

catalyst, washing with methanol (2 x 25 ml.). The filtrate was a solution of 2-n-butoxyethyl p-aminobenzoate.

The intermediate product was not isolated and the filtrate from the first stage was re-charged to the hydrogenator together with 100 ml. 40% formalin, 6 ml. acetic acid, 2 g. of fresh Pd/C paste and 100 ml. methanol. The mixture was then hydrogenated at 490 $KN/m^2$ (70 p.s.i.) and 75-80$^{o}$C for a further 18 hours. After cooling to 55$^{o}$, the mixture was filtered to recover catalyst, washing with methanol (2 x 25 ml.). The filtrate was treated with water (350 ml.) and the precipitated oil extracted into toluene (200 ml.). The lower aqueous was separated and the toluene layer, washed with water (100 ml.) and sodium bicarbonate (ca. 2 g.) until the pH was separated and the toluene layer was treated with activated charcoal (2 g.), stirred at 20 - 25$^{o}$, filtered and stripped to dryness under reduced pressure.

Yield = 97 g.
= 101.5% overall based on p-nitrobenzoic acid.

Assay (g.l.c.) = 94.6%

CLAIMS:

1. A compound of the formula:

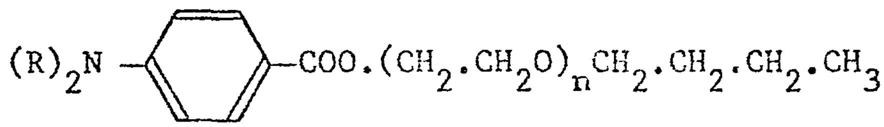

where each R is a methyl or ethyl group and n is 1 or 2.

2. 2-(n-Butoxy)ethyl 4-dimethylaminobenzoate.

3. A photocurable composition comprising a photocurable ethylenically unsaturated compound, a photoinitiator and, as a photoactivator, one or more compounds as claimed in either claim 1 of claim 2.

4. A composition as claimed in claim 3 wherein the photoactivator is present in an amount from 0.2 to 5% by weight of the composition and the photoinitiator is present in an amount from 0.1 to 5% by weight of the composition.

5. A composition as claimed in either claim 3 or claim 4 wherein the photoinitiator is 2-chlorothioxanthone, one or more 2-alkyl-thioxanthones or a mixture thereof.

6. A photoinitiator-photoactivator combination comprising a compound of the formula:

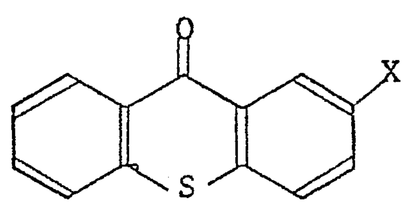

where X is a chlorine atom or an alkyl group in solution in a compound of a mixture of compounds of the formula:

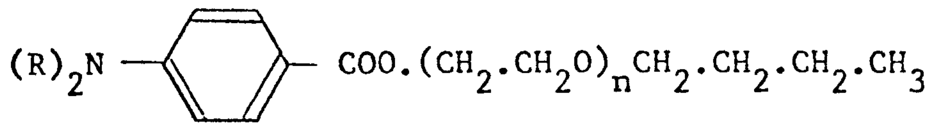

where each R is a methyl or ethyl group and n is 1 or 2.

7. A combination as claimed in claim 6 wherein the weight ratio between the photoactivator and photoinitiator is from 1:4 to 100:1.

8. A composition as claimed in any one of claims 3 to 5 wherein the photoinitiator and photoactivator are provided by a combination as claimed in either claim 6 or claim 7.

9. A method of making a cured plastics composition which comprises exposing a composition as claimed in any one of claims 3 to 5 and 8 to a dose of actinic radiation sufficient to cure it.

0003872

European Patent Office

# EUROPEAN SEARCH REPORT

Application number

EP 79 300 159.5

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| A,D | DE - A - 2 458 345 (BASF)<br>claim<br>--<br>Chemical Abstracts vol. 85, no. 2,1976<br>Columbus, Ohio, USA<br>T. TAKEUCHI et al."Sensitizers for photosensitive resin compositions"<br>pages 475, column 2 and pages 476, column 1, abstract no. 12 359s<br>& JP - A - 75, 158, 323 (TOYO INK)<br>---- | 1<br><br><br><br><br>5 | C 07 C 101/62<br>C 08 F 2/50<br>C 08 K 5/18 |
| | | | TECHNICAL FIELDS SEARCHED (Int.Cl.²)<br>C 07 C 101/62<br>C 08 F 2/50<br>C 08 K 5/18 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family,<br>corresponding document |

| Place of search | Date of completion of the search | Examiner |
|---|---|---|
| Berlin | 29-05-1979 | KNAACK |

EPO Form 1503.1 06.78